# EUROPÄISCHE PATENTSCHRIFT

(11) **EP 2 147 586 B1**
(45) Veröffentlichungstag und Bekanntmachung des Hinweises auf die Patenterteilung: **13.10.2010**
(21) Anmeldenummer: 08707700.4
(22) Anmeldetag: 13.02.2008
(51) Int. Cl.: H05K 9/00, A61B 5/04

(54) **MESSCONTAINER FÜR BIOMAGNETISCHE MESSUNGEN**
MEASUREMENT CONTAINER FOR BIOMAGNETIC MEASUREMENTS
RÉCIPIENT DE MESURE POUR MESURES BIOMAGNÉTIQUES

(30) Priorität: 11.04.2007 DE 102007017316
(43) Veröffentlichungstag der Anmeldung: 27.01.2010
(73) Patentinhaber: BMDSys Production GmbH, 39118 Magdeburg (DE)
(72) Erfinder: KRÜMMEL, Torsten, 6318 Walchwill (CH)
(74) Vertreter: Stößel, Matthias
(86) Internationale Anmeldenummer: PCT/EP2008/001091
(87) Internationale Veröffentlichungsnummer: WO 2008/125160

(56) Entgegenhaltungen:
- EP-A- 0 225 954
- EP-A- 0 381 273
- EP-A- 0 528 301
- DE-A1- 2 316 933

## Beschreibung

### Gebiet der Erfindung

Die Erfindung betrifft einen Messcontainer für biomagnetische Messungen, welcher insbesondere für magnetokardiologische Messungen eingesetzt werden kann. Weiterhin betrifft die Erfindung ein biomagnetisches Messsystem, welches einen erfindungsgemäßen Messcontainer umfasst. Derartige Messcontainer und biomagnetische Messsysteme können insbesondere im Bereich der Kardiologie, jedoch auch in anderen medizinischen Bereichen, wie beispielsweise der Neurologie, eingesetzt werden. Auch andere Anwendungen sind jedoch denkbar.

### Stand der Technik

In den vergangenen Jahren und Jahrzehnten haben magnetische Messsysteme, welche bis dahin im Wesentlichen der Grundlagenforschung vorbehalten waren, Einzug in viele Bereiche der biologischen und medizinischen Wissenschaften gehalten. Insbesondere die Neurologie und die Kardiologie profitieren von derartigen biomagnetischen Messsystemen.

Grundlage der biomagnetischen Messsysteme ist die Tatsache, dass die meisten Zellaktivitäten im menschlichen oder tierischen Körper mit elektrischen Signalen, insbesondere elektrischen Strömen, verbunden sind. Die Messung dieser elektrischen Signale selbst, welche durch die Zellaktivität hervorgerufen werden, ist beispielsweise aus dem Bereich der Elektrokardiographie bekannt. Neben den rein elektrischen Signalen sind die elektrischen Ströme jedoch auch mit einem entsprechenden Magnetfeld verbunden, deren Messung sich die verschiedenen bekannten biomagnetischen Messmethoden zunutze machen.

Während die elektrischen Signale bzw. deren Messung außerhalb des Körpers von verschiedenen Faktoren, wie beispielsweise den unterschiedlichen elektrischen Leitfähigkeiten der Gewebetypen zwischen der Quelle und der Körperoberfläche verbunden sind, durchdringen magnetische Signale nahezu ungestört diese Gewebebereiche. Die Messung dieser Magnetfelder und deren Änderungen erlaubt somit Rückschlüsse auf die innerhalb des Gewebes fließenden Ströme, zum Beispiel elektrische Ströme innerhalb des Herzmuskels. Eine Messung dieser magnetischen Felder mit hoher Zeit- und/oder Ortsauflösung über einen gewissen Bereich hinweg erlaubt somit bildgebende Verfahren, welche beispielsweise eine aktuelle Situation verschiedener Bereiche eines menschlichen Herzens wiedergeben können. Andere bekannte Anwendungen liegen beispielsweise im Bereich der Neurologie.

Die Messung von Magnetfeldern biologischer Proben oder Patienten, bzw. die Messung von zeitlichen Änderungen dieser Magnetfelder, stellt jedoch messtechnisch eine hohe Herausforderung dar. So sind beispielsweise die Magnetfeldänderungen im menschlichen Körper, welche bei der Magnetokardiographie zu messen sind, ungefähr eine Million Mal schwächer als das magnetische Feld der Erde. Die Detektion dieser Änderungen erfordert also extrem sensitive Magnetsensoren. In den meisten Fällen werden daher im Bereich der biomagnetischen Messungen supraleitende Quanten-Interferenz-Messgeräte (Superconducting Quantum Interference Devices, SQUIDs) eingesetzt. Derartige Sensoren müssen in der Regel, um den supraleitenden Zustand zu erreichen bzw. aufrechtzuerhalten, typischerweise auf 4 °K (-269°C) gekühlt werden, wozu üblicherweise flüssiges Helium verwendet wird. Die SQUIDs sind daher in der Regel einzeln oder in einem SQUID-Array in einem sog. Dewar-Gefäß angeordnet und werden dort entsprechend gekühlt. Alternativ werden zur Zeit Laser-.gepumpte magneto-optische Sensoren entwickelt, die annähernd vergleichbare Empfindlichkeit aufweisen können. Auch in diesem Fall werden die Sensoren in der Regel in einer Array-Anordnung in einem Behälter zur Temperaturstabilisierung angeordnet.

Die Messung der extrem schwachen magnetischen Felder bzw. deren Änderungen, welche im Pikotesla- bzw. Sub-Pikoteslabereich liegen, ist naturgemäß extrem empfindlich gegenüber elektromagnetischen und magnetischen Störungen. Die Magnetfelddetektoren, gleich welcher Art, müssen augelesen werden, wofür eine Vielzahl elektronischer Vorrichtungen bekannt ist. Diese Ausleseelektronik reagiert jedoch empfindlich gegenüber eingekoppelten fremden elektromagnetischen Feldern, welche starke Störungen verursachen können. Weitere Störungen ergeben sich durch den starken Signalhintergrund äußerer magnetischer Felder, wie insbesondere Mikropulsationen des Erdmagnetfeldes oder anderer magnetischer Felder, insbesondere zeitlich sich verändernder magnetischer Felder, wie sie in der Industriegesellschaft in vielfältiger Weise hervorgerufen werden (z.B. durch Bewegung großer ferromagnetischer Massen, wie beispielsweise Züge, LKWs etc.).

Aus dem Stand der Technik sind verschiedene Ansätze bekannt, das Problem der Störeinflüsse zu lösen. So beschreibt beispielsweise WO 03/073117 A1 eine der vielen bekannten Vorrichtung zum Messen von Magnetfeldern im Sub-Piloteslabereich. Die Vorrichtung verwendet einen SQUID, welcher induktiv an ein nicht abgeschirmtes Gradiometer gekoppelt ist. Die Vorrichtung umfasst einen Filter zum Filtern von magnetisch oder elektrisch eingekoppelter Radio-Frequenz-Interferenz. Auf diese Weise sollen die Anforderungen an die elektromagnetische Abschirmung der Messvorrichtung gesenkt werden und der Betrieb eines SQUIDs in rauen Umgebungen überhaupt ermöglicht werden.

Die Praxis hat jedoch gezeigt, dass trotz einer verbesserten Eingangsfilterung nach wie vor die Signale biomagnetischer Messungen starken Einflüssen durch äußere elektromagnetische und magnetische Felder unterworfen sein können, da die oben erwähnten Filter nur eine Erleichterung im Betrieb der Sensoren bewirken, aber in der Regel keine Auswirkung auf die elektromagnetischen Störungen im Frequenzbereich der zu messenden biomagnetischen Signale haben. In vielen Fällen wird es daher in der Praxis unumgänglich sein, eine entsprechende Abschirmung gegen die elektromagnetischen und/oder magnetischen Felder vorzusehen. So sind seit langem aus dem zivilen (z.B. medizinischen) und militärischen Bereich Wirbelstromabschirmungen gegen elektromagnetische Wechselfelder bekannt, welche sowohl stationär als auch beweglich ausgestaltet sein können. Gegen niederfrequente Einflüsse werden in der Regel Abschirmungen aus weichmagnetischen Materialien eingesetzt, welche bislang überwiegend stationär installiert werden.

EP 0 359 864 B1 beschreibt eine Einrichtung und ein Verfahren zur Messungen von schwachen, orts- und zeitabhängigen Magnetfeldern. Die Einrichtung umfasst eine Lagerungseinrichtung zur Aufnahme des Untersuchungsobjekts sowie eine Sensoranordnung mit einem SQUID-Array. Weiterhin wird eine magnetische Abschirmkammer beschrieben, welche für magnetische Wechselfelder mit einer Frequenz von 0,5 Hz einen Abschirmfaktor von wenigstens 10, von für magnetische Wechselfelder mit einer Frequenz von 5 Hz einen Abschirmfaktor von wenigstens 100 und für magnetische Wechselfelder mit einer Frequenz von 50 Hz und darüber einen Abschirmfaktor von wenigstens 1000 aufweist. Zudem weist die Abschirmkammer für hochfrequente Wechselfelder (Frequenzen größer als 10 kHz) Abschirmfaktoren von wenigstens 1000 auf.

Die in EP 0 359 864 B1 beschriebene Abschirmkammer ist jedoch in der Praxis außerordentlich aufwändig. Es sind insbesondere aufwändige bauliche Maßnahmen erforderlich, um die Abschirmkammer in ein Gebäude zu integrieren, da ein entsprechender Fundamentsockel vorgesehen sein muss, welcher in der Größenordnung zwischen 10 und 20 t liegt und aus eisenfreiem Beton hergestellt ist. Eine Ortsänderung oder ein Transport der Vorrichtung ist somit in der Praxis nahezu ausgeschlossen.

Ein weiterer Nachteil der in EP 0 359 864 B1 beschriebenen Vorrichtung liegt darin, dass trotz der aufwändigen Abschirmung zahlreiche Verbindungen zwischen dem Innenbereich der Abschirmkammer und dem Außenbereich bestehen, welche beispielsweise durch die Durchführung der Halterung der Patientenliege durch die Bodenabschirmung sowie durch weitere zahlreiche durch die Abschirmung durchgeführte Pfosten und elektrische Durchführungen bedingt sind. Diese Durchführungen bewirken jedoch, dass magnetische und elektromagnetische Felder in das Innere der Abschirmkammer eingekoppelt werden und dort die Messung empfindlich beeinträchtigen können.

EP 0 528 301 beschreibt eine Vorrichtung zur Verbesserung des Abschirmfaktors einer Abschirmkabine, wobei die Abschirmkabine in einem Kabineraum aufgestellt ist, der eine Schale aus weichmagnetischem Material aufweist.

Eine besondere Problematik der bekannten Abschirmkammern besteht weiterhin in der Anordnung der erforderlichen Messelektronik bzw. der für die Auswertung, insbesondere eine Bildauswertung, erforderlichen Computersysteme. Wird die Messelektronik und das Computersystem vollständig bzw. teilweise im Inneren der Abschirmkammer angeordnet, so stören diese die Messungen durch die Elektronik bzw. Computersysteme erzeugten elektromagnetischen Felder. Zudem ist eine Bedienung der Computersysteme während der eigentlichen Messung hierdurch in diesem Fall praktisch nicht möglich, da das Bedienpersonal sich während der Messung nicht in der Abschirmkammer aufhalten sollte, um die Messung nicht zu beeinflussen. Wird die Messelektronik und das Computersystem andererseits außerhalb der Abschirmkammer angeordnet, so sind wiederum Durchführungen erforderlich, über welche elektromagnetische und magnetische Felder in das Innere der Abschirmkammer eingekoppelt werden können.

Eine weitere Problematik derartiger bekannter Abschirmkammern besteht darin, dass insbesondere im Bereich der Magnetokardiographie die Betreuung der Patienten zu jedem Zeitpunkt gewährleistet sein muss. Insbesondere bei Patienten mit schwerwiegenden Herzproblemen ist eine ständige Überwachung sicherzustellen, sowie im Ernstfall auch eine Anwendung Notfallmedizinischer Sofortmaßnahmen, wie beispielsweise einer Defibrillation. Im Inneren einer Abschirmkammer, wie beispielsweise der in EP 0 359 864 B1 beschriebenen Abschirmkammer, ist eine derartige Betreuung jedoch in der Praxis kaum möglich, da beispielsweise die Anwendung eines Defibrillators gleichzeitig auch die Sensorik und/oder die Messelektronik beschädigen würde und so erhebliche Kosten verursachen würde. Zudem fehlt in den meisten Abschirmkammern der für die notfallmedizinische Betreuung erforderliche Raum.

### Aufgabe der Erfindung

Aufgabe der vorliegenden Erfindung ist es daher, eine Abschirmung für biomagnetische Messungen bereitzustellen, welche insbesondere für magnetokardiologische Messungen eingesetzt werden kann. Die Abschirmung soll einerseits eine effiziente Abschirmung gegenüber Hochfrequenzfeldern und vorzugsweise auch gegenüber niederfrequenten magnetischen Feldern bieten, welche beispielsweise die Anforderungen an die Messeelektronik senken soll. Gleichzeitig soll die Abschirmung jedoch einfach zu handhaben, vorzugsweise transportabel und schnell zu integrieren sein, um biomagnetischen Messungen ein weiteres Anwendungsfeld zu eröffnen als dies bislang gegeben ist.

### Beschreibung der Erfindung

Die Aufgabe wird gelöst durch einen Messcontainer mit den Merkmalen des Anspruchs 1. Vorteilhafte Weiterbildungen der Erfindung sind in den abhängigen Ansprüche dargestellt. Die zusätzlichen Merkmale der anhängigen Ansprüche können sowohl einzeln als auch in Kombination miteinander verwirklicht sein.

Es wird dementsprechend vorgeschlagen, die Abschirmung als Messcontainer auszugestalten, im Gegensatz zu den bekannten, üblicherweise in eine Gebäudestruktur integrierten Abschirmungen. Der vorgeschlagene Messcontainer ist für biomagnetische Messungen, insbesondere im Bereich der Magnetokardiologie, jedoch auch in anderen Anwendungsbereichen, einsetzbar und löst die oben beschriebenen Probleme der aus dem Stand der Technik bekannten Abschirmungen zumindest weitgehend. Weiterhin wird ein biomagnetisches Messsystem vorgeschlagen, welches einen erfindungsgemäßen Messcontainer in einer der beschriebenen Ausgestaltungen umfasst.

Unter einem Messcontainer ist dabei ein Container zu verstehen, welcher für sich alleine und vorzugsweise ohne zusätzliche statische Hilfsmittel eine selbsttragende Konstruktion mit einem im Wesentlichen abgeschlossenen und von einer Containerwand umschlossenen Innenraum bereitstellt. Vorzugsweise ist der Container, wie unten näher ausgeführt wird, transportabel ausgestaltet, um einen leichten Ortswechsel zu ermöglichen.

Ähnlich zu der in EP 0 359 864 B1 beschriebenen Ausgestaltung umfasst der vorgeschlagene Messcontainer eine elektromagnetische Außenabschirmung (Wirbelstromabschirmung) gegen elektromagnetische Hochfrequenzfelder und eine elektromagnetische Innenabschirmung gegen Niederfrequenzfelder (ebenfalls eine Wirbelstromabschirmung). Unter Hochfrequenzfeldern werden dabei im Folgenden Felder mit einer Frequenz von mindestens 10 kHz, vorzugsweise von mindestens 100 kHz, bis maximal einige Gigahertz, z.B. 10 GHz, verstanden, unter Niederfrequenzfeldern hingegen Felder mit einer Frequenz von unterhalb von 100 Hz. Auch im Bereich zwischen 100 Hz und 10 kHz kann eine Abschirmwirkung gegeben sein.

Eine grundlegende Idee der vorliegenden Erfindung besteht, im Gegensatz zu EP 0 359 864 B1, darin, den Messcontainer nicht als Einkammersystem auszugestalten, sondern als Zweikammersystem. Dementsprechend umfasst der vorgeschlagene Messcontainer einen Außencontainer mit der Außenabschirmung. In den Außencontainer eingebracht ist eine Messkammer, deren Wand die Innenabschirmung beinhaltet. Zwischen der Innenwand des Außencontainers und der Messkammer ist eine von außen begehbare Vorkammer ausgebildet.

Das vorgeschlagene Zweikammersystem weist gegenüber dem Stand der Technik eine Vielzahl von Vorteilen auf und ermöglicht die Ausweitung biomagnetischer Messsysteme auf zahlreiche neue Anwendungsgebiete. Insbesondere kann mittels des vorgeschlagenen Messcontainers ein biomagnetisches Messsystem aufgebaut sein, wobei in der Messkammer ein magnetisches Sensorsystem aufgenommen ist, insbesondere ein SQUID-System. Beispielsweise kann es sich bei diesem magnetischen Sensorsystem um das in EP 0 359 864 B1 vorgeschlagene Sensorsystem handeln oder um das in WO 03/073117 A1 beschriebene Sensorsystem. Weiterhin kann mit dem magnetischen Sensorsystem eine Messelektronik gekoppelt sein, welche zur Auswertung des SQUID-Systems bzw. zum Auslesen der von diesem generierten Signale eingerichtet ist. Beispielsweise kann hierfür wiederum auf die oben erwähnten Druckschriften verwiesen werden. Ohne Einschränkung können jedoch auch andere Sensorsysteme verwendet werden, wie beispielsweise die oben erwähnten magnetooptischen Sensorsysteme.

Weiterhin kann in der Messkammer eine Objektliege, insbesondere eine Patientenliege aufgenommen sein, auf welcher der Patient bzw. ein anderes zu untersuchendes biologisches Objekt in einer entsprechenden Stellung angeordnete werden kann.

Außerhalb der Messkammer, in der Vorkammer, kann ein Auswertungsrechner aufgenommen sein, welcher von der Messelektronik aufgenommene Messdaten empfangen kann. Zu diesem Zweck können der Auswertungsrechner und die Messelektronik beispielsweise über eine entsprechende Datenübertragung biomagnetische Messdaten austauschen. Insbesondere kann diese Datenübertragung eine optische Faserverbindung umfassen.

Die zweiteilige Aufteilung des Messcontainers ermöglicht also zum einen die Trennung des Auswertungsrechners von der Messelektronik, so dass das magnetische Sensorsystem durch den Auswertungsrechner nicht oder nur unwesentlich gestört wird. Die Zahl der Durchführungen in die Messkammer kann jedoch gering gehalten werden und es können störungsunanfällige optische Faserverbindungen verwendet werden. Gleichzeitig wird der Auswertungsrechner bzw. die Datenübertragung zum Auswertungsrechner durch die Außenabschirmung wirksam gegenüber elektromagnetischen Einflüssen geschützt, welche somit den Datenempfang und die Datenauswertung kaum beeinflussen und gleichzeitig auch nicht in die Durchführungen in die Messkammer einkoppeln können.

Zum anderen ermöglicht, trotz der beschriebenen effizienten Abschirmung, die Aufteilung als Zweikammersystem eine erheblich verbesserte Nähe des Bedienpersonals zum Patienten. Zwar kann das Bedienpersonal, beispielsweise eine medizinisch technische Assistentin, sich während der eigentlichen Messung in der Regel nicht innerhalb der Messkammer aufhalten, da dies die Messergebnisse erheblich stören könnte. Das Bedienpersonal kann sich jedoch in der begehbaren Vorkammer aufhalten, beispielsweise um den Auswertungsrechner zu bedienen und die Messung zu steuern und somit im Notfall leicht und schnell eingreifen zu können, wenn innerhalb der Messkammer ein medizinischer Notfall eintreten sollte. Dies stellt gegenüber heutigen Systemen, bei welchen der Patient vom außerhalb der Abschirmung befindlichen Bedienpersonal üblicherweise durch aufwändige Schleusensysteme getrennt ist, einen erheblichen Fortschritt dar und trägt nicht nur zur Sicherheit sondern auch zum subjektiven Wohlbefinden des Patienten erheblich bei.

Dieser Gedanke kann dadurch weitergeführt werden, dass die Objektliege, insbesondere die Patientenliege, derart eingerichtet ist, dass diese von der Vorkammer in die Messkammer und zurück verbringbar ist. Beispielsweise kann die Patientenliege zu diesem Zweck roll- oder gleitbar ausgestaltet sein. In diesem Fall ist die Vorkammer entsprechend bemaßt, um die Patientenliege vollständig aufnehmen zu können. Diese Weiterbildung hat den Vorteil, dass beispielsweise im Falle eines medizinischen Notfalls der Patient leicht und schnell in die Vorkammer verbracht und dort entsprechend notfallmedizinisch versorgt werden kann, beispielsweise durch Defibrillation, ohne dass das in der Messkammer befindliche magnetische Sensorsystem geschädigt würde.

Der vorgeschlagene Messcontainer und das biomagnetische Messsystem stellen also gegenüber herkömmlichen magnetischen Abschirmungen sowohl hinsichtlich der magnetischen und elektromagnetischen Abschirmung (und damit hinsichtlich der Signalqualität) als auch hinsichtlich der Benutzerfreundlichkeit und des Risikos für den Patienten eine erhebliche Verbesserung dar. Zudem kann auf aufwändige Schleusensysteme und bauliche Baumaßnahmen zur Integration des Messcontainers verzichtet werden, wobei die Signalqualität durch die Minimierung bzw. Optimierung und Ausgestaltung der Durchführungen ins Innere der Messkammer und eine geeignete Anordnung von in der Messkammer benötigten Komponenten und von lediglich in der Vorkammer benötigten Komponenten weiter optimiert werden kann.

Wie oben beschrieben, soll die Außenabschirmung gegen elektromagnetische Hochfrequenzfelder, also Felder oberhalb von 10 kHz, wirksam sein. Unter einer Abschirmung ist dabei allgemein eine Dämpfung der Feldstärke um mindestens einen Faktor 2, vorzugsweise um mindestens einen Faktor 5, zu verstehen. Besonders bevorzugt ist es, wenn die Außenabschirmung gegen elektromagnetische Felder bei Frequenzen zwischen 100 kHz und 800 MHz eine Dämpfung von mindestens 10 dB erzeugt, vorzugsweise von mindestens 40 dB und besonders bevorzugt von mindestens 60 dB. Die Innenabschirmung erzeugt vorzugsweise eine Abschirmung von mindestens 10 dB bei 50 Hz und vorzugsweise eine Abschirmung von mindestens 40 dB.

Weiterhin kann die Innenabschirmung, zusätzlich zur Abschirmung gegen niederfrequente elektromagnetische Felder, eine Magnetabschirmung umfassen. Die Magnetabschirmung kann insbesondere eine weichmagnetische Abschirmung aufweisen, mit einer Abschirmung magnetischer Felder von unterhalb 1 Hz. Dabei ist es besonders bevorzugt, wenn die Abschirmung der magnetischen Felder bei 0,01 Hz einen Faktor von mindestens 5 beträgt, vorzugsweise einen Faktor von 7 bis 9. Dabei hat es sich als besonders vorteilhaft erwiesen, wenn für die Magnetabschirmung weichmagnetische Materialien mit einer Permeabilität von mindestens 100, vorzugsweise von mindestens 1000 und besonders bevorzugt von mindestens 10000, eingesetzt werden. Beispielsweise kann die Magnetabschirmung mindestens einen der folgenden Werkstoffe aufweisen: eine weichmagnetische Eisenlegierung, insbesondere eine weichmagnetische Nickel-Eisen-Legierung, insbesondere eine weichmagnetische Nickel-Eisen-Legierung mit einem Nickelgehalt von 75% bis 80%. Die letztgenannte Legierung ist in der Praxis unter anderem unter der Bezeichnung µ-Metall oder auch Mumetall, oder unter der Bezeichnung Magnifer, kommerziell erhältlich.

Eine weitere vorteilhafte Ausgestaltung der Erfindung betrifft die Verbindung zwischen Vorkammer und dem Außenbereich außerhalb des Messcontainers. Vorzugsweise wird diese Verbindung durch eine in die Wand des Außencontainers eingelassene Tür hergestellt, welche vorzugsweise ebenfalls eine Hochfrequenzabschirmung gegen elektromagnetische Hochfrequenzfelder aufweist. Insbesondere kann die Hochfrequenzabschirmung die oben beschriebenen Abschirmeigenschaften aufweisen.

Zur Verbesserung der Patientenbetreuung kann auch die Messkammer mit der Vorkammer nicht einfach nur durch eine Öffnung oder Klappe verbunden sein, sondern ebenfalls durch eine entsprechende Tür (Messkammertür). Die Messkammer ist vorzugsweise als begehbare Messkammer ausgestaltet, so dass beispielsweise nicht lediglich ein liegender Patient in die Messkammer eingebracht werden kann, sondern dieser beispielsweise auch durch entsprechendes Bedienpersonal vor Ort in der Messkammer (bevor die Messung durchgeführt wird) eingerichtet, instruiert und betreut werden kann. Auch eine notfallmedizinische Betreuung des Patienten kann dadurch sichergestellt werden. Entsprechend zur Tür des Außencontainers kann auch die Messkammertür eine Abschirmung gegen die elektromagnetischen Niederfrequenzfelder, und vorzugsweise auch gegen die magnetischen Felder, aufweisen, beispielsweise ebenfall mit den oben bezüglich der Innenabschirmung beschriebenen Abschirmeigenschaften.

Eine weitere vorteilhafte Weiterbildung der vorliegenden Erfindung besteht darin, den Messcontainer möglichst einfach und kostengünstig auszugestalten und eine Implementierung in ein Gebäude zu erleichtern. Zu diesem Zweck kann auf vorteilhafte Weise der Außencontainer derart ausgestaltet sein, dass dieser nicht nur die beschriebenen Abschirmeigenschaften aufweist, sondern gleichzeitig auch eine tragende, stabilisierende Funktion einnimmt. Der Außencontainer verwirklicht also gleichzeitig die beschriebenen Abschirmfunktionen und eine tragende statische Funktion. Dadurch unterscheidet sich der Messcontainer in dieser vorteilhaften Ausgestaltung beispielsweise von einfachen abschirmenden Auskleidungen abgeschirmter Räume und kann beispielsweise als Stand-Alone-System ausgestaltet sein. Das vorgeschlagene Zweikammersystem bietet dann insbesondere den Vorteil, dass zwei hintereinander liegende und auch hintereinander begehbare Kammern (Vorkammer und das Innere der Messkammer) bereitgestellt werden, die im Sinne der Abschirmung ineinander verschachtelt sind, wobei vorzugsweise die Vorkammer so konstruiert ist, dass diese durch ihre Länge einen wesentlichen Beitrag zur Entlastung der durch den Messcontainer beaufschlagten Bodenfläche leistet.

Vorzugsweise kann der Außencontainer einen Schichtaufbau aufweisen, mit mindestens einer nicht-metallischen Schicht und mindestens einer metallischen Schicht. Beispielsweise kann die nicht-metallische Schicht mindestens einen der folgenden Werkstoffe aufweisen: einen Polymerschaum, insbesondere einen Polyurethanschaum; einen Polymerwerkstoff, insbesondere eine Polymerplatte, insbesondere eine PVC-Platte; einen Holzwerkstoff, insbesondere eine Holzspanplatte; einen Mineralfaserwerkstoff; einen Glasfaserwerkstoff; einen Harzlaminat-Werkstoff, insbesondere einen faserverstärkten Harzlaminat-Werkstoff.

Besonders bevorzugt ist es dabei, wenn der Schichtaufbau einen Sandwich-Aufbau umfasst. So können beispielsweise eine Innenplatte, ein Füllmaterial und eine Außenplatte vorgesehen sein. Dabei sind vorzugsweise die Innenplatte und/oder die Außenplatte aus einem metallischen Werkstoff hergestellt bzw. umfassen einen derartigen metallischen Werkstoff. Aus Kosten- und Gewichtsgründen sowie aus Gründen der einfachen Verarbeitung bietet sich hier insbesondere Aluminium als eine Schicht des Sandwich-Aufbaus an, da Aluminium gute elektromagnetische Abschirmeigenschaften aufweist und gleichzeitig leicht zu verarbeiten ist. Auch ohne Verwirklichung des Gedankens des Sandwich-Aufbaus kann Aluminium vorteilhaft für den Außencontainer bzw. auch zumindest teilweise für die Messkammer eingesetzt werden. Alternativ oder zusätzlich können jedoch auch Kupfer oder Legierungen eingesetzt werden. Auch andere metallische Werkstoffe sind einsetzbar.

Das Füllmaterial des Sandwich-Aufbaus kann insbesondere zu der oben beschriebenen vorteilhaften tragenden Funktion des Außencontainers beitragen. So kann das Füllmaterial beispielsweise eine Holzkonstruktion aufweisen. Beispielsweise kann der Sandwich-Aufbau derart ausgestaltet sein, dass eine Holzkonstruktion, beispielsweise eine Konstruktion aus Dachlatten eines Weichholzes, zu einem entsprechenden Rahmen zusammengefügt wird. Anschließend wird diese Holzkonstruktion vorteilhafterweise mit einem weiteren Füllmaterial ausgefüllt. Beispielsweise kann hierfür die Holzkonstruktion mit einem Polyurethanschaum oder einem anderen der oben beschriebenen Füllmaterialien oder einer Kombination der Füllmaterialien, ausgefüllt werden. Nach Aufbringen der Innenplatte und der Außenplatte (wobei diese Begriffe auch jeweils eine Kombination mehrerer Platten umfassen können) wird hieraus der beschriebene Sandwich-Aufbau gebildet.

Auf diese Weise kann ein kostengünstiger Messcontainer hergestellt werden, welcher den mechanischen Anforderungen an die Stabilität einer freitragenden Konstruktion genügt und gleichzeitig eine effiziente Abschirmung bewirkt, ohne dass aufwändige Maßnahmen seitens der Gebäudegestaltung zur Aufnahme des Messcontainers erforderlich sind.

In Kombination mit der oben beschriebenen Sandwich-Konstruktion, jedoch auch mit anderen Ausgestaltungen des Außencontainers, hat es sich als vorteilhaft erwiesen, wenn die Innenabschirmung und/oder die Außenabschirmung mindestens eines der folgenden Metalle aufweist: Aluminium, Kupfer, eine Aluminiumlegierung; eine Kupferlegierung. Insbesondere kann die Außenabschirmung eine metallische Schicht mit einer Dicke von 0,5 mm bis 10 mm, insbesondere von 1 mm bis 5 mm, aufweisen. Die Innenabschirmung weist vorzugsweise eine metallische Schicht mit einer Dicke von mindestens 5 mm, vorzugsweise von mindestens 8 mm, auf.

Die Messkammer kann insbesondere, im Gegensatz zu früheren bekannten Abschirmkammern, nicht-konzentrisch im Außencontainer aufgenommen sein. Die Lage der Messkammer innerhalb des Außencontainers hat sich in der Praxis als unwesentlich herausgestellt, so dass die Aufteilung in Messkammer und Vorkammer entsprechend der übrigen Anforderungen, beispielsweise den Platzanforderungen an die Vorkammer, vorgenommen werden kann. Beispielsweise kann auf diese Weise die Aufnahme einer Patientenliege innerhalb der Vorkammer sichergestellt sein.

Eine weitere vorteilhafte Weiterbildung der vorliegenden Erfindung berücksichtigt die Tatsache, dass eine mangelhafte elektrische Verbindung der Außenabschirmung und der Innenabschirmung eines Innenraums bzw. im vorliegenden Fall der Messkammer zu elektrisch unerwünschten Effekten führen kann. In einer vorteilhaften Weiterbildung der Erfindung ist daher der Zwischenraum zwischen der Innenwand des Außencontainers und der Messkammer zumindest teilweise mit einem leitfähigen Material ausgefüllt ist, derart, dass eine elektrisch leitfähige Verbindung zwischen der Innenabschirmung und der Außenabschirmung erzeugt wird. Auf diese Weise wird die Außenabschirmung im Bereich der Messkammer elektrisch gesehen Bestandteil der Innenabschirmung, mit nichtvernachlässigbaren Vorteilen auch in Bezug auf das Abschirmverhalten bei niedrigen Frequenzen. Beispielsweise kann die Messkammer im Bereich einer Unterseite der Messkammer, der Oberseite der Messkammer und einer Rückwand der Messkammer über das leitfähige Material mit der Innenwand des Außencontainers verbunden sein. In diesem Fall wäre beispielsweise lediglich die der Vorkammer zugewandte Vorderseite der Messkammer nicht in Kontakt mit dem leitfähigen Material. Auch andere Ausgestaltungen sind jedoch denkbar.

Als besonders vorteilhaft hat es sich dabei erwiesen, wenn das leitfähige Material ein verformbares leitfähiges Material aufweist. Beispielsweise kann es sich hierbei um ein plastisch und/oder elastisch verformbares leitfähiges Material handeln. Auch Materialien, welche lediglich in einen ersten Zustand verformbar sind, anschließend jedoch aushärten, wie beispielsweise leitfähige Epoxide, sind möglich. Besonders bevorzugt ist es, wenn das verformbare leitfähige Material eine Leitpaste umfasst. Auch die Verwendung einer Silberleitpaste oder anderer mit leitfähigen Partikeln gefüllter Pasten oder Flüssigkeiten sind denkbar. Vorzugsweise soll in Zusammenhang mit der Verwendung von Aluminium als Basismaterial für die Abschirmung die Pasten oder Flüssigkeiten Chemikalien beinhalten, die das Problem des Aluminiumoxids lösen.

Wie oben aufgeführt, zeichnet sich der vorgeschlagene Messcontainer insbesondere durch seinen einfachen und kostengünstigen Aufbau aus. So kann insbesondere der Außencontainer gleichzeitig die Funktion der elektromagnetischen Abschirmung und die tragende Funktion übernehmen. Beispielsweise kann der Außencontainer bezüglich seiner mechanischen Belastbarkeit gängigen Container-Standards entsprechen, beispielsweise dem ISO 1496-2-Standard. Um einen schnellen Transport sicherzustellen und um beispielsweise die Integration des Containers in bestehende Räumlichkeiten zu erleichtern, kann der Messcontainer insbesondere als transportabler Messcontainer ausgestaltet sein und Transportelemente (zum Beispiel entsprechende Ösen oder anderer aus der Containertechnologie bekannte Transportelemente) aufweisen, welche den Eingriff einer Transportvorrichtung ermöglichen.

Weitere Einzelheiten und Merkmale der Erfindung ergeben sich aus der nachfolgenden Beschreibung eines bevorzugten Ausführungsbeispiels in Verbindung mit den Unteransprüchen. Hierbei können die jeweiligen Merkmale für sich alleine oder zu mehreren in Kombination miteinander verwirklicht sein. Die Erfindung ist nicht auf das Ausführungsbeispiel beschränkt. Das Ausführungsbeispiel ist in den Figuren schematisch dargestellt. Gleiche Bezugsziffern in den einzelnen Figuren bezeichnen dabei gleiche oder funktionsgleiche bzw. hinsichtlich ihrer Funktion einander entsprechende Elemente.

Im Einzelnen zeigt:
- Figur 1: eine Schnittdarstellung eines Ausführungsbeispiels eines erfindungsgemä- ßen biomagnetischen Messsystems mit einem erfindungsgemäßen Messcon- tainer in Ansicht von oben;
- Figur 2: das biomagnetische Messsystem gemäß Figur 1 in Schnittdarstellung von der Seite; und
- Figur 3: eine Vergrößerung des in Figur 2 mit A bezeichneten Ausschnitts der Wand des Messcontainers.

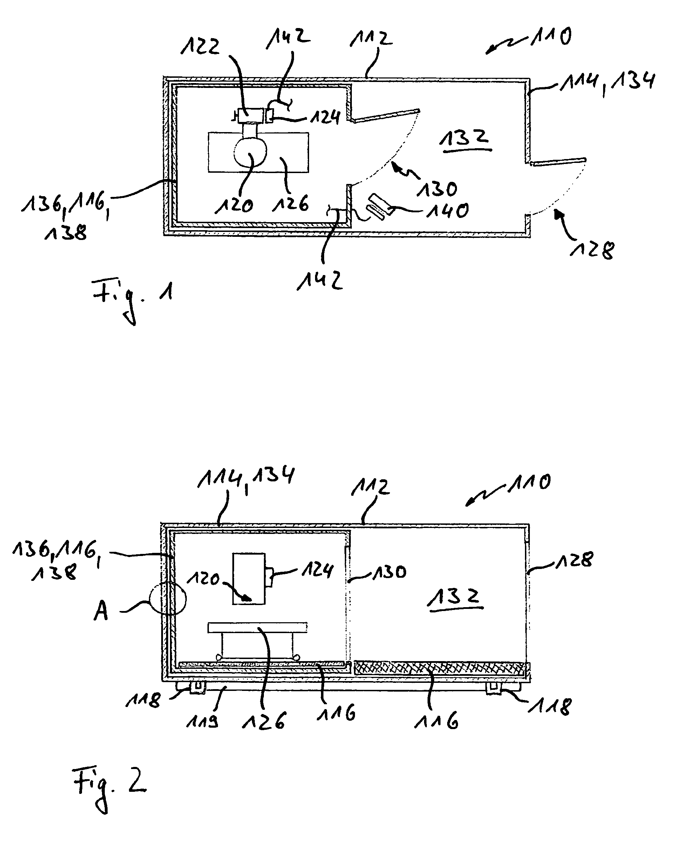

In den Figuren 1 und 2 ist ein Ausführungsbeispiel eines erfindungsgemäßen biomagnetischen Messsystems 110 dargestellt. Das biomagnetische Messsystem 110 umfasst ein Ausführungsbeispiel eines erfindungsgemäßen Messcontainers 112, welcher in Figur 1 in Schnittdarstellung in Ansicht von oben und in Figur 2 in Schnittdarstellung in Ansicht von der Seite dargestellt ist.

Der Messcontainer 112 ist erfingdungsgemäß als Zweikammersystem aufgebaut und umfasst einen Außencontainer 114, in welchen eine Messkammer 116 eingebracht ist.

Der Außencontainer 114 kann beispielsweise als Standard-Container ausgebildet sein und kann beispielsweise auf einen Lkw geladen, dort befestigt und auf diese Weise transportiert werden. Zu diesem Zweck können beispielsweise am Außencontainer 114 entsprechende Transportelemente 118 vorgesehen sein, welche dem Fachmann bekannt sind. Beispielsweise kann es sich dabei um Standard-Transportelemente handeln, mittels derer der Messcontainer 112 angehoben bzw. auf einem Lastwagen, einem Güterwagen oder einem Schiff fixiert werden kann. Beispielsweise können vier derartiger Transportelemente 118 vorgesehen sein. Des Weiteren dient der Außencontainer 114 dem Zweck der Gewichtsverteilung, um das Gesamtgewicht des Messcontainers 112 und der darin aufgenommenen Geräte auf eine entsprechende Fläche zu verteilen.

Die Transportelemente 118, welche in Figur 2 lediglich schematisch dargestellt sind, sind im vorliegenden Ausführungsbeispiel Bestandteile einer Unterbodenkonstruktion 119, welche unterhalb des Außencontainers 114 angeordnet ist. Beispielsweise kann diese Unterbodenkonstruktion einen Aluminiumrahmen umfassen, welcher mit dem Außencontainer 114 durch dem Fachmann bekannte Befestigungsmaßnahmen verbunden ist. Beispielsweise kann eine Befestigung durch wenige Schrauben erfolgen, z.B. acht bis zwölf Schrauben. Die Unterbodenkonstruktion kann beispielsweise einen standardisierten Transport auf einem Schiff oder einem LKW ermöglichen, kann Gabelstaplertaschen für den Eingriff von Staplergabeln umfassen, kann bei vorübergehender Lagerung beispielsweise steckbare Füße aufnehmen und kann zur Gewichtsverteilung des Messcontainers 112 beitragen.

Soll der Messcontainer 112 schließlich stationär integriert werden, beispielsweise innerhalb eines Gebäudes auf einem Betonsockel oder einer anderen Bodenstruktur, so kann die Unterbodenkonstruktion 119 vorzugsweise mit wenigen Handgriffen entfernt werden, beispielsweise durch Lösen der Verbindungsschrauben. Dann kann der Messcontainer 112 ohne die Unterbodenkonstruktion 119 beispielsweise wiederum durch Verschrauben und/oder durch andere Befestigungsmittel wie Bolzen, durch Kleben, Schweißen oder Einbetonieren, mit dem Gebäude oder mit einer speziellen, von dem Gebäude getrennten Beton- oder Fundamentplatte verbunden werden.

In dem Messcontainer 112 sind verschiedene weitere Elemente des biomagnetischen Messsystems aufgenommen. So umfasst das biomagnetische Messsystem 110 insbesondere ein magnetisches Sensorsystem 120, welches an einem entsprechenden Gestell 122 vertikal verschiebbar bzw. positionierbar aufgenommen ist. Das magnetische Sensorsystem 120 wird in diesem Ausführungsbeispiel gebildet durch eine Vielzahl von supraleitenden Magnetsensoren (SQUIDs), welche in einem (in den Figuren lediglich angedeuteten) Dewar aufgenommen sind. Dieser Dewar wiederum ist am Gestell 122 in seiner Höhe verstellbar aufgehängt. Ähnlich würde es sich verhalten, wenn das Sensorsystem 120 durch eine Vielzahl von magnetooptischen Sensoren in einem temperierten Behälter gebildet würde.

Mit dem magnetischen Sensorsystem 120 ist auf bekannte Weise eine Messelektronik 124 gekoppelt, welche in Figur 1 ebenfalls lediglich angedeutet ist. Wie oben beschrieben, können das magnetische Sensorsystem 120 und die Messelektronik 124 beispielsweise ausgestaltet sein, wie im Stand der Technik aufgeführt, z.B. wie in WO 03/073117 A1 beschrieben. Auch andere Ausgestaltungen des Sensorsystems 120 und der Messelektronik 124 sind jedoch denkbar und sind dem Fachmann bekannt.

Unterhalb des magnetischen Sensorsystems 120 befindet sich innerhalb der Messkammer 116 eine Patientenliege 126. Diese Patientenliege 126 ist vorzugsweise aus nichtmagnetischem Material hergestellt und ist (was in Figur 2 lediglich symbolisch angedeutet ist) beweglich gelagert. Auf dieser Patientenliege 126 kann ein liegender Patient derart unterhalb des magnetischen Sensorsystems 120 positioniert werden, dass sein Brustbereich vom magnetischen Sensorsystem 120 erfasst wird. Auf diese Weise können magnetokardiographische Abbildungen des Herzens des Patienten durchgeführt werden. Für andere Arten biomagnetischer Messsysteme können entsprechend andere Arten von Objektliegen oder ähnlichen "Probenhalterungen" eingesetzt werden.

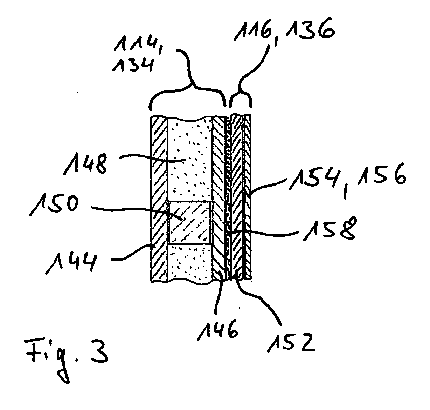

Der Messcontainer 112 in dem in den Figuren dargestellten Ausführungsbeispiel weist zwei Türen auf: eine Tür 128 des Außencontainers und eine Messkammertür 130. Ein Benutzer des biomagnetischen Messsystems 110, beispielsweise ein Patient und/oder Bedienpersonal, betritt den Messcontainer 112 durch die Tür 128 und gelangt hierdurch in eine Vorkammer 132. Die Vorkammer 132 ist vom Bereich außerhalb des Messcontainers 112 lediglich getrennt durch die Wand des Außencontainers 114, welche eine Außenabschirmung 134 aufweist. Diese Außenabschirmung 134 dient, wie oben erläutert und wie anhand von Figur 3 nachstehend weiter ausgeführt wird, der Wirbelstromabschirmung gegenüber elektromagnetischen Hochfrequenzfeldern.

Von der Vorkammer 132 aus betritt der Benutzer dann durch die Messkammertür 130 das Innere der Messkammer 116. Die Messkammerwand der Messkammer 116 umfasst eine Innenabschirmung 136, welche das Innere der Messkammer 116, zusätzlich zur Außenabschirmung 134, gegen elektromagnetische Einflüsse im niederfrequenten Bereich schützt. Wie oben beschrieben, handelt es sich bei dieser Innenabschirmung 136 um eine Wirbelstromabschirmung im niederfrequenten Bereich unterhalb von 100 Hz.

Somit ist das Innere der Messkammer 116 durch die Abschirmungen 134, 136 in unterschiedlichen Frequenzbereichen doppelt gegenüber elektromagnetischen Störungen geschützt. Zusätzlich umfasst die Innenabschirmung 136, wie ebenfalls anhand von Figur 3 unter näher ausgeführt wird, weiterhin eine Magnetabschirmung 138 in Form einer weichmagnetischen Abschirmung. Dementsprechend ist das Innere der Messkammer 116 sowohl gegen hochfrequente als auch gegen niederfrequente elektromagnetische Einstrahlungen als auch gegenüber extrem langsam veränderlichen magnetischen Einflüssen geschützt.

Die Vorkammer 132, welche eine in den Figuren 1 und 2 erkennbare lang gestreckte Gestalt aufweist, bewirkt, dass sogar bei geöffneten Türen 128, 130 lediglich Einstrahlungen aus einem vergleichsweise kleinen Raumwinkel in das Innere des Messcontainers 112 gelangen können, so dass Störungen durch elektromagnetische und magnetische Einflüsse zusätzlich durch die Geometrie des Messcontainers 112 unterdrückt werden. Im Idealfall, in welchem die Vorkammer 132 eine (in den Figuren 1 und 2 in horizontaler Richtung) unendlich lange Ausdehnung hätte, wäre dieser Raumwinkel, unter welchem Störungen durch die geöffneten Türen 128, 130 eindringen könnten, sogar auf einen verschwindend kleinen Raumwinkel reduziert.

Die Zahl der Durchführungen, über welche die Vorkammer 132 mit dem Inneren der Messkammer 116 verbunden ist, kann auf ein absolut notwendiges Minimum reduziert werden. Insbesondere kann die in der Messkammer 116 aufgenommene Messelektronik 124 mit weiterer, in der Vorkammer 132 aufgenommener Messelektronik (nicht dargestellt) und/oder einem Auswertungsrechner 140 (lediglich in Figur 1 dargestellt) durch eine optische Faserverbindung 142 (lediglich in Figur 1 teilweise dargestellt) verbunden sein und Messdaten austauschen. Die Einkopplung elektromagnetischer und/oder magnetischer Störsignale durch Durchführungen in der Wand der Messkammer 116 bzw. der Innenabschirmung 138 können dadurch auf ein Minimum reduziert werden. Eine Energieversorgung der Messelektronik 124 kann beispielsweise durch abgeschirmte Steckverbinder erfolgen, beispielsweise durch abgeschirmte Steckverbinder, welche zusätzliche Filter beinhalten und welche in den Figuren ebenfalls nicht dargestellt sind.

Wie oben beschrieben, stellt die doppelte Abschirmung in Form der Außenabschirmung 134 und der Innenabschirmung 136 ein wesentliches Element des erfindungsgemäßen Messcontainers 112 dar. Um diese Abschirmungen 136, 138 im weiteren Detail zu erläutern, ist in Figur 3 der Ausschnitt, welcher in Figur 2 mit A bezeichnet ist, in vergrößerter Darstellung gezeigt. Dabei ist der Ausschnitt A derart gewählt, dass dieser einen Bereich umfasst, in welchem die Wand der Messkammer 116, und somit die Innenabschirmung 136, in unmittelbarer Nähe zur Wand des Außencontainers 114 und somit zur Außenabschirmung 134 angeordnet sind.

In dem in Figur 3 dargestellten bevorzugten Ausführungsbeispiel ist die Wand des Außencontainers 114 in einem Sandwich-Aufbau hergestellt. Dieser Sandwich-Aufbau setzt sich zusammen aus einer Außenplatte 144 aus faserverstärktem Harzlaminat und einer Innenplatte 146. Zum Zwecke der Bereitstellung der Außenabschirmung 134 in Form einer Wirbelstromabschirmung gegen hochfrequente elektromagnetische Strahlung (Frequenzen oberhalb von 10 kHz) ist die Innenplatte 146 in diesem Ausführungsbeispiel aus einem gut leitenden Material hergestellt. Insbesondere bieten sich hierfür, wie oben beschrieben, als Materialien Kupfer- und/oder Aluminiumplatten oder Metallplatten mit Legierungen der genannten Elemente an. Auch andere gut leitende metallische Materialien sind jedoch einsetzbar. Die Dicke der Innenplatte 146 liegt vorzugsweise zwischen 1 und 5 mm.

Der Zwischenraum zwischen den beiden Platten 144, 146 ist in der in Figur 3 dargestellten Sandwich-Konstruktion im Wesentlichen mit einem formstabilen Füllmaterial 148 ausgefüllt, beispielsweise wiederum mit einem faserverstärkten Harz und/oder mit einem Polymerschaum, beispielsweise einem Polyurethanschaum. Weiterhin ist in den Zwischenraum zwischen den Platten 144, 146 in diesem Ausführungsbeispiel eine Holzkonstruktion 150 eingebracht, welche zur Stabilität des Außencontainers 114 erheblich beiträgt. Die Holzkonstruktion 150 kann jedoch auch durch eine erhöhte Stabilität der Platten 144, 146 und/oder des Füllmaterials 148 ersetzt bzw. ergänzt werden.

Es sei darauf hingewiesen, dass der Bereich A in Figur 2 ein Wandbereich des Außencontainers 114 ist. Die Konstruktion des Außencontainers 114 kann jedoch an verschiedenen Bereichen variieren, so dass beispielsweise im Deckenbereich des Außencontainers 114, im Bodenbereich und im Bereich der Seitenwände unterschiedliche Konstruktionen eingesetzt werden. Insbesondere im Bodenbereich kann die Außenwand des Außencontainers 114 beispielsweise verstärkt ausgestaltet sein und kann für eine gleichmäßige Gewichtsverteilung sorgen und als Gewichtsverteilungselement dienen.

Der Außencontainer 114 übernimmt somit in diesem Fall durch die Innenplatte 146 aus gut leitendem Material, insbesondere aus Aluminium, die Funktion der Außenabschirmung 134 gegen elektromagnetische Hochfrequenzfelder. Weiterhin ist in Figur 3 der Aufbau der Wand der Messkammer 116 dargestellt, welche die Innenabschirmung 136 übernimmt.

Zur Gewährleistung der Innenabschirmung 136 weist die Wand der Messkammer 116 zunächst eine dicke Platte 152 auf, welche der Wand des Außencontainers 114 zuweist. Die Dicke dieser Platte 152 beträgt vorzugsweise mindestens 8 mm. Somit trägt diese Platte 152 wesentlich zur Stabilität der Messkammer 116 bei und ist zumindest teilweise für deren Stabilität verantwortlich.

Zur Bereitstellung der Innenabschirmung 136 gegenüber niederfrequenten elektromagnetischen Störungen können ebenfalls wiederum Kupfer oder Aluminium als Materialien eingesetzt werden. Auch Legierungen sind wiederum einsetzbar, wobei beispielsweise Kupfer oder Aluminium mit einer geringeren Menge an Zusätzen eingesetzt werden können, beispielsweise mit weniger als 4 % einlegierten Zusätzen. Eine Platte 152 mit den beschriebenen Eigenschaften stellt eine wirksame Innenabschirmung 136 mit den oben beschriebenen Eigenschaften im Niederfrequenzbereich dar.

Weiterhin weist die Wand der Messkammer 116 in dem in Figur 3 dargestellten bevorzugten Ausführungsbeispiel eine Magnetabschirmung 154 auf. Dabei dient die Platte 152 als tragende Struktur für den Einbau dieser Magnetabschirmung 154. Zu diesem Zweck ist auf der dem Innenraum der Messkammer 116 zugewandten Seite der Platte 152 eine Platte 156 eines weichmagnetischen Materials aufgebracht. Beispielsweise kann, wie oben beschrieben, zu diesem Zweck eine µ-Metall-Platte eingesetzt werden, beispielsweise in einer Dicke von ca. 1 bis 2 mm. Auch andere weichmagnetische Materialien 156 sind jedoch einsetzbar.

Als weitere Besonderheit weist das in Figur 3 dargestellte bevorzugte Ausführungsbeispiel der Abschirmungen 134, 136 eine großflächige elektrische Verbindung der beiden Wirbelstromabschirmungen (Innenplatte 146 und Platte 152) auf. Zu diesem Zweck ist der Zwischenraum zwischen dem Außencontainer 114 und der Messkammer 116 in dem dargestellten Bereich weitgehend ausgefüllt mit einem elektrisch leitfähigen Füllmaterial 158. Dieses elektrisch leitfähige Füllmaterial 158 stellt eine elektrische Verbindung zwischen den beiden Abschirmungen 134, 136 her, so dass es im Bereich der Messkammer 116 zu einer Verstärkung der Abschirmleistung auch im niederfrequenten Bereich kommt.

Als Beispiel für ein derartiges elektrisch leitfähiges Füllmaterial 158 sind Leitpasten zu nennen. Besonders bevorzugt sind dabei Leitpasten, welche in der Lage sind, isolierende Oberflächen-Oxidschichten abzutragen oder zu durchdringen, um den elektrischen Kontakt zwischen den Platten 146 und 152 zu verbessern. Insbesondere macht sich dies bei der Verwendung von Aluminium bemerkbar, welches dazu neigt, eine Oxidschicht an der Oberfläche auszubilden, welche den elektrischen Kontakt zwischen den Platten 146, 152 verschlechtern würde. Als besonders geeignet für das elektrisch leitfähige Füllmaterial 158 haben sich beispielsweise Pasten oder Flüssigkeiten erwiesen, welche elektrisch leitfähige Partikel, beispielsweise Zinkpartikel, enthalten, welche diese Oxidschicht durchdringen (beispielsweise durch mechanischen Abrieb und/oder durch elektrochemischen Abbau der Oxidschicht) und dadurch den elektrischen Kontakt verbessern. Derartige elektrisch leitfähige Füllmaterialien 148 mit antioxidierender Wirkung werden beispielsweise unter der Bezeichnung "Noalox®" von der Firma Ideal Industries GmbH vertrieben. Auch andere elektrisch leitfähige Füllmaterialien mit vorzugsweise antioxidierender oder Oxidschichtabtragender Wirkung sind jedoch einsetzbar.

Die Verbindung zwischen der Messkammer 116 und dem Außencontainer 114 kann durch übliche Verbindungen erfolgen, beispielsweise durch Verschrauben, durch Bolzen, durch Schweißen, durch Kleben, durch Steckkonstruktionen oder durch andere, dem Fachmann bekannte Ausgestaltungen. Die Konstruktion der Türen 128 und 130 kann im Wesentlichen der Konstruktion der übrigen Wand des Außencontainers 114 bzw. der Messkammer 116 entsprechen, kann jedoch auch einfacher gestaltet werden. Beispielsweise kann die Messkammertür 130 lediglich mit einer Wirbelstromabschirmung in Form der Platte 152 versehen sein, wobei auf die weichmagnetische Abschirmung 156 verzichtet wird.

Der Außencontainer 114 entspricht mit dem in Figur 3 dargestellten Sandwich-Aufbau beispielsweise dem Aufbau üblicher Kühlcontainer. Dabei kann ein Kühlcontainer beispielsweise derart modifiziert werden, dass die üblicherweise als Innenplatte 146 verwendete faserverstärkte Harzlaminat-Platte durch die die Außenabschirmung 134 gewährleistende metallische Innenplatte 146 ersetzt wird. Auf diese Weise ist eine Umrüstung kommerziell erhältlicher Container leicht und schnell realisierbar. Die Unterbodenkonstruktion des Außencontainers 114, welche vorzugsweise ein nicht-ferromagnetisches Material aufweist, kann insbesondere derart ausgestaltet sein, dass Standardvorschriften erfüllt werden, damit der Messcontainer 112 anstelle eines Standardcontainers beispielsweise auf einen Lastkraftwagen geladen, dort befestigt und transportiert werden kann und andererseits die Erfordernisse eines Gewichtsverteilungselements erfüllt.

Im Inneren der Vorkammer 132 und der Messkammer 116 ist auf dem Boden ein Arbeitsboden 116 aus einem Kunststoffmaterial aufgenommen. Dieser Arbeitsboden 116 kann beispielsweise derart ausgestaltet sein, dass dieser die Niveauunterschiede zwischen der Vorkammer 132 und dem Inneren der Messkammer 116 ausgleicht. Dadurch kann beispielsweise gewährleistet sein, dass die Patientenliege 126 vom Inneren der Messkammer 116 in die Vorkammer 132 verbracht werden kann, ohne dass hierbei eine Schwelle überwunden werden muss. Die Vorkammer 132 ist dementsprechend vorzugsweise derart dimensioniert, dass diese die Patientenliege 126 vollständig aufnehmen kann. Auf diese Weise kann die Patientenliege 126 beispielsweise im Falle eines medizinischen Notfalls schnell in die Vorkammer 132 verbracht werden, um dort intensivmedizinische Maßnahmen durchzuführen, wie beispielsweise eine Defibrillation, welche im Inneren der Messkammer 116 die Messelektronik 124 und/oder das magnetische Sensorsystem 120 schädigen könnte.

Der Arbeitsboden 160 kann beispielsweise aus einem Kunststoffmaterial gefertigt sein, beispielsweise einem PVC-Laminat. Je nach Anwendung kann der Arbeitsboden 160 auch zusätzliche Eigenschaften aufweisen, beispielsweise leitfähige Eigenschaften, beispielsweise zur Verhinderung von elektrostatischen Beschädigungen der Messelektronik 124 durch einen Benutzer. Der Arbeitsboden 160 kann beispielsweise mit der Wand des Außencontainers 114 und/oder der Messkammer 116 durch Bolzen, Schrauben, Kleben oder ähnliche Verbindungstechniken verbunden sein.

### Bezugszeichenliste

- 110: biomagnetisches Messsystem
- 112: Messcontainer
- 114: Außencontainer
- 116: Messkammer
- 118: Transportelement
- 119: Unterbodenkonstruktion
- 120: magnetisches Sensorsystem
- 122: Gestell
- 124: Messelektronik
- 126: Patientenliege
- 128: Tür des Außencontainers
- 130: Messkammertür
- 132: Vorkammer
- 134: Außenabschirmung
- 136: Innenabschirmung
- 138: Magnetabschirmung
- 140: Auswertungsrechner
- 142: optische Faserverbindung
- 144: Außenplatte
- 146: Innenplatte
- 148: Füllmaterial
- 150: Holzkonstruktion
- 152: Platte aus leitendem Material
- 154: Magnetabschirmung
- 156: weichmagnetisches Material
- 158: elektrisch leitfähiges Füllmaterial
- 160: Arbeitsboden

## Patentansprüche

1. Messcontainer (112) für biomagnetische Messungen, insbesondere für magnetokardiologische Messungen, umfassend eine Außenabschirmung (134) und eine Innenabschirmung (136) gegen niederfrequente elektromagnetische Felder, wobei der Messcontainer (112) als Zweikammersystem aufgebaut ist, mit einem die Außenabschirmung (134) umfassenden Außencontainer (114), wobei in den Außencontainer (114) eine Messkammer (116) mit der Innenabschirmung (136) eingebracht ist, wobei zwischen der Innenwand des Außencontainers (114) und der Messkammer (116) eine von außen begehbare Vorkammer (132) ausgebildet ist, **dadurch gekennzeichnet, dass** die Außenabschirmung eine Abschirmung gegen elektromagnetische Hochfrequenzfelder darstellt.

2. Messcontainer (112) nach dem vorhergehenden Anspruch, wobei die Außenabschirmung (134) bei Frequenzen zwischen 100 kHz und 800 MHz eine Dämpfung von mindestens 10 dB erzeugt, vorzugsweise von mindestens 40 dB und besonders bevorzugt von mindestens 60 dB.

3. Messcontainer (112) nach einem der beiden vorhergehenden Ansprüche, wobei die Innenabschirmung (136) eine Abschirmung von mindestens 10 dB bei 50 Hz erzeugt, vorzugsweise eine Abschirmung von mindestens 40 dB.

4. Messcontainer (112) nach einem der vorhergehenden Ansprüche, wobei die Innenabschirmung (136) zusätzlich eine Magnetabschirmung (138) umfasst.

5. Messcontainer (112) nach einem der vorhergehenden Ansprüche, wobei die Vorkammer (132) von außen durch eine in die Wand des Außencontainers (114) eingelassene Tür (128) begehbar ist, wobei die Tür (128) vorzugsweise eine Wirbelstromabschirmung gegen elektromagnetische Hochfrequenzfelder aufweist.

6. Messcontainer (112) nach einem der vorhergehenden Ansprüche, wobei die Messkammer (116) eine Messkammertür (130) zur Vorkammer (132) aufweist, um ein Messobjekt, insbesondere einen Patienten, in die Messkammer (116) einzubringen.

7. Messcontainer (112) nach dem vorhergehenden Anspruch, wobei die Messkammertür (130) eine Wirbelstromabschirmung gegen elektromagnetische Niederfrequenzfelder aufweist.

8. Messcontainer (112) nach einem der vorhergehenden Ansprüche, wobei die Wand des Außencontainers (114) einen Schichtaufbau mit mindestens einer nichtmetallischen Schicht (144, 148, 150) und mindestens einer metallischen Schicht (146) aufweist.

9. Messcontainer (112) nach dem vorhergehenden Anspruch, wobei die Wand des Außencontainers (114) einen Sandwichaufbau aufweist, mit einer Innenplatte (146), einem Füllmaterial (148, 150) und einer Außenplatte (144), wobei die Innenplatte (146) und/oder die Außenplatte (144) einen metallischen Werkstoff, insbesondere eine Aluminiumplatte und/oder eine Kupferplatte, aufweisen.

10. Messcontainer (112) nach einem der beiden vorhergehenden Ansprüche, wobei die Innenabschirmung (136) und/oder die Außenabschirmung (134) mindestens eines der folgenden Metalle aufweisen: Aluminium; Kupfer; eine Aluminium-Legierung; eine Kupfer-Legierung.

11. Messcontainer (112) nach einem der vorhergehenden Ansprüche, wobei die Messkammer (116) nicht-konzentrisch in dem Außencontainer (114) aufgenommen ist.

12. Messcontainer (112) nach einem der vorhergehenden Ansprüche, wobei der Zwischenraum zwischen der Innenwand des Außencontainers (114) und der Messkammer (116) zumindest teilweise mit einem leitfähigen Material (158) ausgefüllt ist, derart, dass eine elektrisch leitfähige Verbindung zwischen der Innenabschirmung (136) und der Außenabschirmung (134) erzeugt wird.

13. Messcontainer (112) nach dem vorhergehenden Anspruch, wobei das leitfähige Material (158) ein verformbares leitfähiges Material, insbesondere eine elektrisch leitfähige Paste oder Flüssigkeit, aufweist, insbesondere mindestens eines der folgenden Materialien: eine Silberleitpaste; eine antioxidierende Paste, insbesondere eine mit leitfähigen Metallpartikeln gefüllte Paste.

14. Messcontainer (112) nach einem der vorhergehenden Ansprüche, wobei der Messcontainer (112) als transportabler Messcontainer (112) ausgestaltet ist und Transportelemente (118) für den Eingriff einer Transportvorrichtung und/oder einer Sicherungsvorrichtung aufweist.

15. Biomagnetisches Messsystem (110), umfassend einen Messcontainer (112) nach einem der vorhergehenden Ansprüche, wobei in der Messkammer (116) ein magnetisches Sensorsystem (120) aufgenommen ist, insbesondere ein SQUID-System und/oder ein magneto-optisches System, sowie mindestens eine mit dem magnetischen Sensorsystem (120) gekoppelte Messelektronik (124), wobei in der Vorkammer (132) eine Auswertungsrechner (140) aufgenommen ist, wobei der Auswertungsrechner (140) und die Messelektronik (124) eingerichtet sind, um über eine Datenübertragung (142) biomagnetische Messdaten auszutauschen.

## Claims

1. Measuring container (112) for biomagnetic measurements, in particular for magnetocardiological measurements, comprising an outer screen (134) and an inner screen (136) against low frequency electromagnetic fields, the measuring container (112) being constructed as a two-chamber system with an outer container (114) comprising the outer screen (134), a measuring chamber (116) with the inner screen (136) being introduced into the outer container (114), an antechamber (132) which can be walked into from outside being formed between the inner wall of the outer container (114) and the measuring chamber (116), **characterized in that** the outer screen represents a screen against high-frequency electromagnetic fields.

2. Measuring container (112) according to the preceding claim, in which at frequencies between 100 kHz and 800 MHz the outer screen (134) produces an attenuation of at least 10 dB, preferably at least 40 dB and, with particular preference, at least 60 dB.

3. Measuring container (112) according to one of the two preceding claims, in which the inner screen (136) produces a screening of at least 10 dB at 50 Hz, preferably a screening of at least 40 dB.

4. Measuring container (112) according to one of the preceding claims, in which the inner screen (136) additionally comprises a magnetic screen (138).

5. Measuring container (112) according to one of the preceding claims, in which the antechamber (132) can be walked into from outside by a door (128) let into the wall of the outer container (114), the door (128) preferably having an eddy current screen against high-frequency electromagnetic fields.

6. Measuring container (112) according to one of the preceding claims, in which the measuring chamber (116) has a measuring chamber door (130) to the antechamber (132) in order to introduce a measurement object, in particular a patient, into the measuring chamber (116).

7. Measuring container (112) according to the preceding claim, in which the measuring chamber door (130) has an eddy current screen against electromagnetic low frequency fields.

8. Measuring container (112) according to one of the preceding claims, in which the wall of the outer container (114) has a layered construction with at least one non-metallic layer (144, 148, 150) and at least one metallic layer (146).

9. Measuring container (112) according to the preceding claim, in which the wall of the outer container (114) has a sandwich construction with an inner plate (146), a fill material (148, 150) and an outer plate (144), the inner plate (146) and/or the outer plate (144) having a metallic material, in particular an aluminium plate and/or a copper plate.

10. Measuring container (112) according to one of the two preceding claims, in which the inner screen (136) and/or the outer screen (134) have at least one of the following metals: aluminium; copper; an aluminium alloy; a copper alloy.

11. Measuring container (112) according to one of the preceding claims, in which the measuring chamber (116) is held non-concentrically in the outer container (114).

12. Measuring container (112) according to one of the preceding claims, in which the empty space between the inner wall of the outer container (114) and the measuring chamber (116) is at least partially filled up with a conductive material (158) in such a way that an electrically conductive connection is produced between the inner screen (136) and the outer screen (134).

13. Measuring container (112) according to the preceding claim, in which the conductive material (158) has a deformable conductive material, in particular an electrically conductive paste or liquid, in particular at least one of the following materials: a silver conducting paste; an anti-oxidizing paste, in particular a paste filled with conductive metal particles.

14. Measuring container (112) according to one of the preceding claims, in which the measuring container (112) is configured as a transportable measuring container (112) and has transport elements (118) for the intervention of a transport device and/or a safety device.

15. Biomagnetic measuring system (110) comprising a measuring container (112) according to one of the preceding claims, in which are held in the measuring chamber (116) a magnetic sensor system (120), in particular a SQUID system, and/or a magneto-optical system, as well as at least one electronic measuring system (124) coupled to the magnetic sensor system (120), an evaluation computer (140) being held in the antechamber (132), the evaluation computer (140) and the measurement electronics (124) being set up in order to exchange biomagnetic measured data via a data transmission means (142).

## Revendications

1. Caisson (112) pour mesures biomagnétiques, en particulier pour mesures magnéto-cardiologiques,
comprenant un blindage extérieur (134) et un blindage intérieur (136) contre les champs électromagnétiques à basse fréquence,
le caisson de mesure (112) étant configuré comme système à deux chambres avec un caisson extérieur (114) qui comprend le blindage extérieur (134),
une chambre de mesure (116) dotée du blindage intérieur (136) étant prévue à l'intérieur du caisson extérieur (114),
une pré-chambre (132) à laquelle on peut accéder depuis l'extérieur étant formée entre la paroi intérieure du caisson extérieur (114) et la chambre de mesure (116), **caractérisé en ce que**
le blindage extérieur est un blindage contre les champs électromagnétiques à haute fréquence.

2. Caisson de mesure (112) selon la revendication précédente, dans lequel le blindage extérieur (134) exerce une atténuation d'au moins 10 dB, de préférence d'au moins 40 dB et de façon particulièrement préférable d'au moins 60 dB des fréquences comprises entre 100 kHz et 800 MHz.

3. Caisson de mesure (112) selon l'une des deux revendications qui précèdent, dans lequel le blindage intérieur (136) assure à 50 Hz un blindage d'au moins 10 dB et de préférence un blindage d'au moins 40 dB.

4. Caisson de mesure (112) selon l'une des revendications précédentes, dans lequel le blindage intérieur (136) comprend en outre un blindage magnétique (138).

5. Caisson de mesure (112) selon l'une des revendications précédentes, dans lequel on peut accéder à la pré-chambre (132) depuis l'extérieur par une porte (128) ménagée dans la paroi du caisson extérieur (114), la porte (128) présentant de préférence un blindage par courants de Foucault contre les champs électromagnétiques à haute fréquence.

6. Caisson de mesure (112) selon l'une des revendications précédentes, dans lequel la chambre de mesure (116) présente une porte (130) vers la pré-chambre (132) pour placer dans la chambre de mesure (116) un objet à mesurer et en particulier un patient.

7. Caisson de mesure (112) selon la revendication précédente, dans lequel la porte (130) de la chambre de mesure présente un blindage par courants de Foucault contre les champs électromagnétiques à basse fréquence.

8. Caisson de mesure (112) selon l'une des revendications précédentes, dans lequel la paroi du caisson extérieur (114) présente une structure stratifiée qui présente au moins une couche non métallique (144, 148, 150) et au moins une couche métallique (146).

9. Caisson de mesure (112) selon la revendication précédente, dans lequel la paroi du caisson extérieur (114) présente une structure stratifiée avec une plaque intérieure (146), un matériau de charge (148, 150) et une plaque extérieure (144), la plaque intérieure (146) et/ou la plaque extérieure (144) présentant un matériau métallique, en particulier une plaque d'aluminium et/ou une plaque de cuivre.

10. Caisson de mesure (112) selon l'une des deux revendications qui précèdent, dans lequel le blindage intérieur (136) et/ou le blindage extérieur (134) présentent au moins l'un des métaux suivants : aluminium, cuivre, alliage d'aluminium et alliage de cuivre.

11. Caisson de mesure (112) selon l'une des revendications précédentes, dans lequel la chambre de mesure (116) est reprise de manière non concentrique dans le caisson extérieur (114).

12. Caisson de mesure (112) selon l'une des revendications précédentes, dans lequel l'espace intermédiaire entre la paroi intérieure du caisson extérieur (114) et la chambre de mesure (116) est remplie au moins en partie d'un matériau conducteur (158) de manière à former une liaison électriquement conductrice entre le blindage intérieur (136) et le blindage extérieur (134).

13. Caisson de mesure (112) selon la revendication précédente, dans lequel le matériau conducteur (158) présente un matériau conducteur déformable, en particulier une pâte ou un liquide électriquement conducteur, et en particulier au moins l'un des matériaux suivants : une pâte conductrice à l'argent, une pâte antioxydante et en particulier une pâte chargée de particules métalliques conductrices.

14. Caisson de mesure (112) selon l'une des revendications précédentes, dans lequel le caisson de mesure (112) est configuré comme caisson de mesure (112) transportable et présente des éléments de transport (118) qui permettent de le saisir par un dispositif de transport et/ou un dispositif de fixation.

15. Système (110) de mesure biomagnétique comprenant un caisson de mesure (112) selon l'une des revendications précédentes, un système (120) de détecteurs magnétiques, en particulier un système SQUID et/ou un système magnéto-optique, étant repris dans la chambre de mesure (116) de même qu'au moins une électronique de mesure (124) raccordée au système (120) de détecteurs magnétiques, un calculateur d'évaluation (140) étant repris dans la pré-chambre (132), le calculateur d'évaluation (140) et l'électronique de mesure (124) étant conçus pour échanger des données de mesure biomagnétiques par transfert (142) de données.
